# EUROPEAN PATENT APPLICATION

(11) **EP 3 311 914 A1**
(43) Date of publication of application: **25.04.2018**
(21) Application number: 16813780.0
(22) Date of filing: 21.06.2016
(51) Int. Cl.: B01J 20/28, G01N 33/18, C04B 38/08

(54) **PASSIVE CERAMIC SAMPLER FOR MEASURING WATER CONTAMINATION**

(30) Priority: 22.06.2015 ES 201530882
(71) Applicant: Consejo Superior de Investigaciones Cientificas (CSIC), 28006 Madrid (ES)
(72) Inventor: LACORTE BRUGUERA, Silvia, 08034 Barcelona (ES); FRANQUET GRIELL, Helena, 08034 Barcelona (ES); SILVA TREVIÑO, Jorge, 50009 Zaragoza (ES); ORERA CLEMENTE, Victor Manuel, 50009 Zaragoza (ES)
(74) Representative: Pons Ariño, Angel
(86) International application number: PCT/ES2016/070467
(87) International publication number: WO 2016/207461

(57) **Abstract**

The invention relates to a passive ceramic sampler for measuring water contamination, comprising a porous ceramic casing and an adsorbent filler material. The casing has a porous structure combining mechanical stiffness with good permeation to solutions and retention of the adsorbent filler material. The invention also relates to the method for the production of the sampler, as well as to the use of same for the detection of contaminants, such as pesticides, cytostatics, polycyclic aromatic hydrocarbons or plasticisers.

## Description

### OBJECT OF THE INVENTION

The object of the present invention relates to a passive ceramic sampler for measuring water contamination, comprising a porous ceramic casing and an adsorbent filler material.

The casing has a porous structure combining mechanical stiffness with good permeation to solutions and retention of the adsorbent filler material.

### STATE OF THE ART

Passive samplers are systems that enable the preconcentration of contaminants dissolved in water and the objective of which is determining the chemical quality of the water.

Passive sampling is the integrated gathering of a sample during a certain period by means of the free flow of contaminants in the medium into the receiving system, which depends on the sampling device. The movement of the analytes is carried out due to the differences between the chemical potentials outside and inside the sampler until the state of equilibrium or until the end of the sampling. Passive sampling for organic contaminants in the aquatic medium has been described previously [Gorecki et al.,(2002), Trends Anal. Chem. 21:276-291]. The system is based on the sorption of the contaminants on a receiving phase, and the performance thereof depends on the polarity and solubility of the contaminants and on the type of water to be monitored.

Passive samplers are essentially applied to the control of contaminants in surface water and groundwater with the aim of obtaining information integrated with time or in hard-to-access areas where it is difficult to gather or pump water.

There are a wide variety of passive samplers:
- **Dialysis membranes:** these passive collectors were the first to be used and are particularly useful for monitoring metals. They enable the migration of the metals from the water to the hydrophobic receiving phase (generally hexane) through a dialysis membrane. The main problem with these devices is that the dialysis membrane is hydrophilic, which limits the diffusion of the more hydrophobic compounds.
- **Empore® Disks:** have been used so far as an extraction technique, however due to the high surface to volume relationship thereof, they can be used as a sampling device in the field. The most used phase is C18 but other phases are commercially available.
- **Ceramic dosimeters:** they are ceramic cylinders filled with an adsorbent capable of retaining different families of organic contaminants such as polycyclic aromatic hydrocarbons (PAHs), benzene, toluene, ethylbenzene or xylene (BTEX) or chlorinated hydrocarbons in groundwater [Bopp et al. (2005), J. Chromatogr A 1072:137-147].
- **Polar Organic Chemical Integrative Sampler (POCIS):** It enables estimating the accumulative exposure of lingering organic contaminants and the average concentrations during a time period of exposure. It enables monitoring a wide range of contaminants, among others, to control estrogenic compounds in river water. [Mazzella, N. et al. (2010), Environmental Science and Technology 44 (5): 1713-1719].
- **Semipermeable membrane devices (SPMD):** a hydrophobic membrane (usually polyethylene) is used with a specific hydrophobic phase that enables retaining the non-polar contaminants present in the medium. These are tubular polyethylene membranes containing a lipid film, such as triolein, which is capable of accumulating the organic contaminants present in water. SPMDs can be standardised and used over long periods; they can detect very low concentrations of organic contaminants and mimic the concentration of contaminants that accumulate in organisms. SPMDs have possibly been the most widely used devices for determining organic contaminants in environmental samples. However, it does not enable detecting polar compounds since the membranes are impermeable to these compounds or the accumulation is thermodynamically unfavourable due to the low affinity of the receiving phase for these contaminants.
- **Diffusive gradients in thin-films (DGT):** this system enables diffusion of the analyte through a membrane filter and a gel layer before being retained in the receiving phase. The accumulation is based on a concentration gradient and therefore it is a dynamic sampler that depends on a flow that is proportional to a concentration. However, the thickness of the diffusing gel layer controls the transfer of mass of the contaminants, such that in practice the sampling rate is relatively independent of the water flow [Chen et al.(2013), Environmental Science and Technology, 47:13587-13593]. This system has been successfully applied to determine antibiotics in wastewater and soil [Chen et al. (2015), Talanta, 132: 902-908].

Both patent DE 198 30 413 and the article by P. Grathwohl et al. (2001) in the Journal of Process Analytical Chemistry 6-2: 68-73 disclose a ceramic dosimeter for taking samples of contaminants in surface water and groundwater. The possibility of using multiple designs for the ceramic or for the filler is envisaged. Specifically, in said patent (see claim 4) it is indicated that the pore size is comprised between 3 and 1000 nm with a porosity (see claim 5 in DE 198 30 413) comprised between 30% and 50%. The article offers a specific example in which the pore size is 5 nm and the adsorbent used is IRA-Amberlite.

Finally, Addeck A. et al. (2011), Organohalogen Compounds 73, 2108-2111, discloses a ceramic toximeter with a porosity of 30%, with an X-CARB filler, without indicating a specific pore size and Cristale J. et al. (2013), Environmental Pollution 172 : 163-169 describes a ceramic sampler with a pore size of 5 nm and the use of a Sigma - Aldrich hydrophilic polymer filling (HLB: hydrophilic lipophilic balance), capable of retaining a wide range of organic compounds from an aqueous matrix.

### DESCRIPTION OF THE INVENTION

The object of the present invention is a passive ceramic sampler for measuring water contamination, comprising a porous ceramic casing and an adsorbent filler material. The ceramic casing has a microstructure comprising:
- a structural skeleton made of ceramic material in which spheroidal cavities with a diameter comprised between 10 and 15 µm are inserted, connected by windows smaller than 1 µm through which the liquid is permeated.
- aggregates of the ceramic material that do not have cavities, but do have pores with sizes comprised between 20 and 90 nm.

In a particular embodiment, the casing has a geometry that is selected from among cylindrical, spherical, prismatic, ellipsoidal, toroidal, frustoconical or truncated-pyramidal. In a preferred embodiment, the casing has a cylindrical geometry, with a length comprised between 10 and 300 mm, a diameter comprised between 2 and 100 mm and a thickness comprised between 1 and 5 mm, the ceramic material being alumina.

Particularly, the microstructure has an axial heterogeneity, the density of the cavities with a diameter comprised between 10 and 15 µm decreasing from the outside to the inside. In no case does the microstructure of the casing have connected pores with a diameter smaller than 20 nm.

In both bases of the sampler with cylindrical geometry, caps are placed in order to prevent the outlet of adsorbent material contained on the inside.

Another aspect of the present invention constitutes a method for the production of the ceramic sampler comprising the following stages:
- preparing a dust suspension of the ceramic material with sizes comprised between 0.24 and 0.49 µm and water in proportions comprised between 60% and 80% by volume.
- adding a pore-forming agent to the suspension in proportions comprised between 12% and 18% by volume and a dispersant with 1% by weight of the solid.
- pouring the suspension prepared in the previous stages into a plaster matrix with the geometry of the desired final piece
- maintaining the suspension in the plaster matrix during a period of time comprised between 10 and 20 minutes
- sintering the ceramic material according to a cycle that in turn comprises:
   - heating from room temperature to sintering temperature in a range between 1300 and 1500 °C with a slope of 4°C/min
   - maintaining at the sintering temperature for 3 hours
   - cooling to room temperature with a slope of 4°C/min

Another aspect of the present invention is the use of the passive ceramic sampler for detecting contaminants dissolved in water.

In particular embodiments, the contaminants can be pesticides, cytostatics, polycyclic aromatic hydrocarbons or plasticisers.

### BRIEF DESCRIPTION OF THE FIGURES

**Fig. 1****:** Diagram of the use of the ceramic sampler in which
   1. Ceramic dosimeter
   2. Contaminants
   3. Water flow
   4. Polymer adsorbent
   5. Extraction
   6. Analysis of contaminants by chromatography
**Fig. 2****:** Sintering cycle of the ceramic casing
**Fig. 3****:** Open pore ΔV normalized to the weight of the sample based on the pore size
**Fig. 4****:** Optical microscopy image (x50) of the inner portion of the ceramic casing showing the area with cavities and the elongated particles of nanoporous alumina.
**Fig. 5****:** Optical microscopy image (x100) of the portion close to the outside of the ceramic casing showing the area with cavities and the nanoporous alumina particles.
**Fig. 6****:** SEM micrograph of the area with cavities showing the small holes that connect the cavities and give the porosimetry measurements.
**Fig. 7****:** SEM micrograph of the outer portion of the casing showing the differences in microstructure (density of the cavities) between the surface and inner area and of the casing.

### DETAILED DESCRIPTION OF THE INVENTION

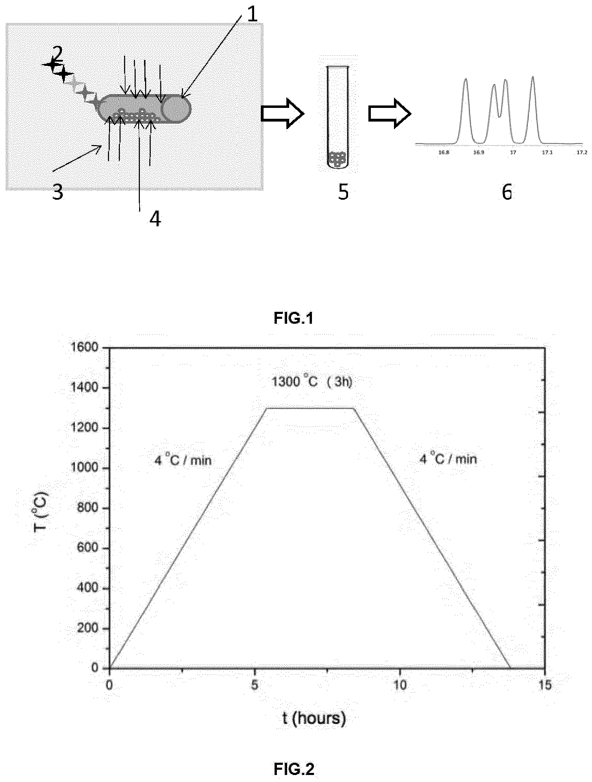

As already indicated in previous sections, the novelty of the ceramic sampler with respect to the state of the art lies in the microstructure of the ceramic casing, which allows elevated diffusivity of the analytes and therefore enables increasing the load capacity of the contaminants in the receiving phase. The other novelty of the system is that it is filled with an adsorbent polymer capable of retaining polar and non-polar compounds (of an elevated octanol-water partition coefficient, Kow) and therefore, enables carrying out screenings of environmental contaminants with very distant physical and chemical properties. Still another novelty of the system is that the dimensions of the ceramic device can be varied so that it can be adapted to the type of water to be analysed, such that waters with a high content of organic matter can be analysed with smaller systems while, in very pristine areas, where the concentration capacity has to be high, a large system can be used in order to facilitate the inlet and retention of the analytes in the receiving phase. In Figure 1, the designed device and the use protocol are diagrammed. The ceramic samplers object of the present invention possess the following properties:
- **Effectiveness:** they enable determining the contaminants at ng levels by means of the application thereof in the sampling area for periods ranging from days to 1-3 weeks of sampling.
- **Integrity and stability:** they enable determining the presence of contaminants in a certain area in a time-integrated manner, such that the different contaminants present in a mass of water can be identified during a certain period of time. Furthermore, the contaminants, once adsorbed on the adsorbent, do not degrade and therefore, the system enables detecting at trace levels any contaminant present in the water.
- **Capacity:** the sampler is characterised by having a great retention capacity, which allows preconcentration or accumulation of an elevated number of contaminants and it is also capable of retaining elevated concentrations.
- **Versatility:** due to the capacity of the polymer-based adsorbent to accumulate both polar or non-polar compounds, the passive sampler can be used for determining a large number of contaminants with different physical and chemical properties. Thus, the system is able to identify contaminants that, by means of occasional sampling, could easily "escape" and not be identified.
- **Durability:** they are systems that can be reused up to 3 times without losing effectiveness.
- **Autonomy:** these are systems that do not require electricity for the operations thereof nor any other energy source, since the operations thereof are based only on the passive diffusion of the contaminants from the water to the adsorbent.
- **Legal adequacy:** by means of the incubation of the samplers for 1 week, the system is able to detect regulated contaminants at levels below the maximum environmental quality levels imposed by European legislation.
- **Cost:** they are very inexpensive systems because the cost of the ceramic cylinder and the adsorbent required for the operations thereof is less than 4 €, which means that they can be used for the environmental monitoring of a highly elevated number of contaminants and reduce the costs associated with conventional sampling.

The passive sampler enables the application thereof in different types of water and therefore is characterised by the versatility and applicability thereof for the control of different water masses. Due to the microstructure thereof, the sampler enables an elevated flow of water and enables a large mass of contaminants to be accumulated in a relatively short period of time (one week). On the other hand, being a stable system, it can also be used during long periods of time. Some of the intended applications are:
- **Monitoring of priority contaminants:** the objective is to use the system for monitoring the presence of regulated contaminants in surface waters (rivers, lakes, ponds) in order to implement European Directives on the chemical quality of water masses.
- **Monitoring of emerging contaminants:** In order to identify non-priority contaminants according to current legislation, the device enables identifying "unknown" compounds that are not expected to be found in water but that are potentially toxic contaminants for the environment or harmful to human health. These contaminants can be found in environmental waters due to spillage, effluents from sewage treatment plants, uncontrolled effluents, runoffs, etc.
- **Spill Control:** in areas with industrial (automotive, textile, pharmaceutical, tanning, food, plastic, dyes etc.) and hospital spills, the objective is to identify the presence of specific contaminants that could affect the quality of the water, especially if spilled into the sewer network since they could affect the waste water treatment plant (WWTPs) which would affect regular operations of a WWTP and therefore, the quality of the surface waters where the effluents are spilled.
- **Control of effluents from the WWTPs (waste water treatment plants):** many contaminants are recalcitrant and are not removed during the biological treatment system of the WWTPs, such that the effluents from treatment plants become a source of contaminants for the environment. The type of influent and the characteristics of each treatment plant define treatment effectiveness and therefore the load of contaminants spilled into the environment. By means of the control of treatment plant effluents, the aim is to identify the most ubiquitous contaminants and thus design tertiary treatment systems necessary for the complete removal thereof.
- **Quality control of distribution water:** water purification systems vary according to the quality of the catchment water and the size of the supply. Purification systems can be a simple chlorination or use highly sophisticated systems based on primary treatment, filtration and ultrafiltration and osmosis. In order to protect human health against possible poisoning due to the presence of organic contaminants, continuous monitoring of water quality is necessary. The use of samplers in the outlets of the water treatment plants enables controlling water quality, such that the quality thereof can be guaranteed.

### EMBODIMENT OF THE INVENTION

### Description of the embodiment

The method for the production of an alumina sampler with cylindrical geometry and having the following dimensions is described:
- length: 45 mm
- outer diameter: 13 mm
- inner diameter: 10 mm
- thickness: between 2 and 3 mm

The ceramic casing is manufactured by slip casting by pouring a colloidal suspension into a porous plaster matrix with the geometry of the desired end piece. The suspension is made up of 0.5 microns alumina dust and water. Furthermore, pore-former agent (for example, corn flour) and a dispersant (for example Duramax D-3005) are added as additives to facilitate the stability of the suspension. The dwell time of the suspension in the matrix determines the thickness of the green body and the composition of the suspension, the porosity and microstructure of the piece.

The green body is sintered at high temperature in air according to the cycle shown in Figure 2. A piece with high stiffness and mechanical resistance but with high porosity is thus obtained.

### Structural characteristics of the ceramic sampler

The typical porosity for this type of system is between 25 and 50%. In the example device, total porosity is measured though density and is 50%. Open porosity is measured by means of mercury porosimetry and is 34.5%.

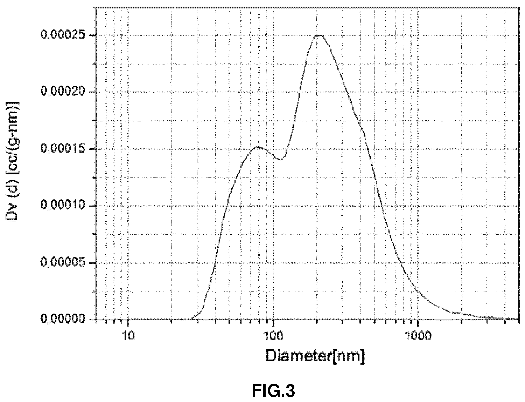

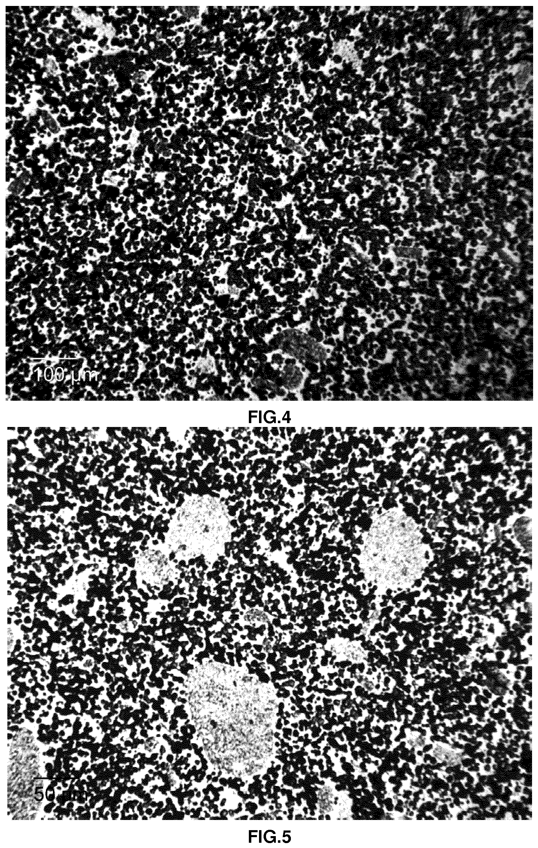

The size of the holes for the passage of liquids is reflected in Figures 3 and 4, from which it follows that:
- there are no open pores with a diameter smaller than 20 nm
- the size distribution of the windows connecting cavities is markedly bimodal with two characteristic regions:
   - most of the windows have diameters between 200 and 500 nm.
   - another group of nanopores in the aggregates of ceramic material has sizes comprised between 20 and 90 nm in diameter.

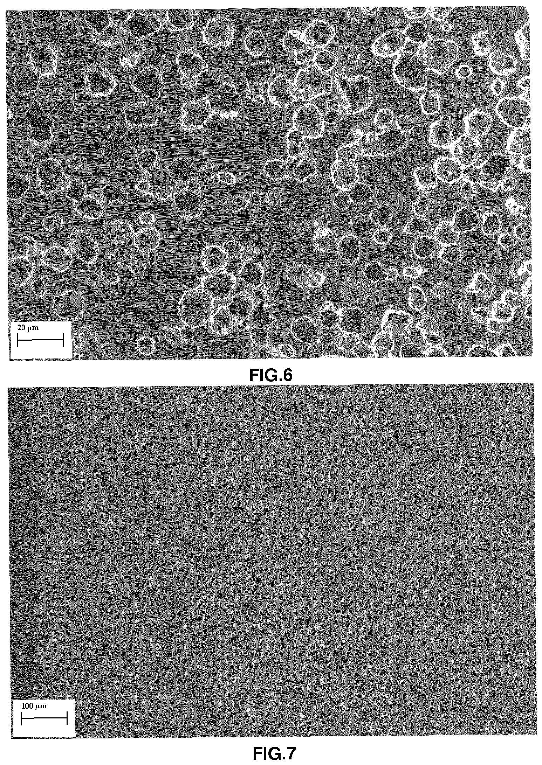

The microstructure of the ceramic is shown in Figures 4 - 7. A continuous area is observed with spheroidal cavities of about 10-15 µm in diameter connected by windows smaller than the micron embedded in a continuous frame of alumina with pores that are not observable under the optical microscope (nanopore). This nanoporous region has typical thicknesses of about 20 microns. In SEM (Figures 6 and 7) it can be seen that this pore is small, smaller than 1 µm.

Large alumina aggregates with small pores and without cavities can also be seen. It is important to note that the distribution of the cavities decreases from the outside to the inside. Near the inner wall, in an area of about 100-200 µm, the density thereof is lower.

This microstructure is a result of the production process and allows high permeability to liquids while preventing leakage of the adsorbent polymers.

### Results in detecting contaminants

Four families of contaminants have been analysed, which are characterised by the ubiquity thereof in the environment.

Organochlorine pesticides are compounds used in agriculture and in Spain they have been synthesised for several decades. As a result, it is a family of compounds that is ubiquitous in the environment and, due to the persistence and toxicity thereof, they must be controlled according to the Water Framework Directive (39/2013/EU) in surface waters.

Cytostatics are compounds that are used to treat cancer and have recently been identified as a new family of environmental contaminants.

Endocrine disruptors, represented by the 3 most ubiquitous compounds in water, are compounds used as detergents and plasticisers and they have the capacity to affect the hormonal system of organisms. Nonylphenol and octylphenol are covered by Directive 39/2013/EU while bisphenol A is not regulated but is a substance of elevated production according to ECHA-REACH (Regulation (CE) No. 1907/2006 of the European Parliament and of the Council) which is the European Regulation concerning the Registration, Evaluation, Authorisation and Restriction of Chemicals.

Finally, some Polycyclic Aromatic Hydrocarbons (PAHs) were studied, with special emphasis on naphthalene since it is widely used and explicitly included in Directive 39/2013/EU, as they are potentially carcinogenic compounds. The different compounds studied are indicated in table 1, where the amounts of ng accumulated after an exposure period of 6 days are indicated.

**Table 1. List of compounds studied and accumulation (in ng) of contaminants in the dosimeter after an exposure period of 6 days.**

| **Compounds** | **ng accumulated (6 days)** | **Analytical technique*** |
|---|---|---|
| *Organochlorine pesticides* | | |
| α-HCH | 40 | GC-MS |
| βHCH | 11 | GC-MS |
| γHCH (lindane) | 25 | GC-MS |
| δHCH | 14 | GC-MS |
| Hexachlorobenzene | 10 | GC-MS |
| 2,4'-DDD | 12 | GC-MS |
| 4,4'-DDD | 3 | GC-MS |
| 2,4'-DDE | 5 | GC-MS |
| 4,4'-DDE | 5 | GC-MS |
| 2,4'-DDT | 25 | GC-MS |
| 4,4'-DDT | 10 | GC-MS |

| *Cytostatic compounds* | | |
|---|---|---|
| Chlorambucil | 8 | LC-MS/MS |
| Ifosfamide | 2 | LC-MS/MS |
| Cyclophosphamide | 24 | LC-MS/MS |
| Capacitabine | 13 | LC-MS/MS |
| Irinotecan | 1 | LC-MS/MS |
| Daunorubicin | 1 | LC-MS/MS |
| Prednisone | 15 | LC-MS/MS |
| Cytarabicine | 1 | LC-MS/MS |
| Doxorubicin | 5 | LC-MS/MS |

| *Endocrine disruptors* | | |
|---|---|---|
| Bisphenol A | 668 | GC-MS |
| Nonylphenol | 643 | GC-MS |
| Octylphenol | 116 | GC-MS |

| *Polycyclic aromatic hydrocarbons* | | |
|---|---|---|
| Naphthalene | 265 | GC-MS |
| Acenaphthene | 61 | GC-MS |
| Fluorene | 13 | GC-MS |
| Phenanthrene | 12 | GC-MS |
| Anthracene | 15 | GC-MS |

| | | |
|---|---|---|
| • GC-MS= gas chromatography coupled to mass spectrometry; LC-MS/MS=liquid chromatography coupled to mass spectrometry in tandem. | | |

200 mg of OASIS HLB adsorbent is weighed and conditioned with methanol, which is decanted, and water. The cylindrical ceramic sampler is filled with the adsorbent, being careful not to reach the volume limit of the cylinder. The cap is placed, and it is left in a horizontal position in order to prevent the adsorbent from depositing on one end.

The sampler is hung inside a beaker with Milli-Q water, holding it in the centre, and is spiked with a standard. They are prepared in duplicate and are left in stirring for a certain time (1, 3 and 6 days for example), covered from the light in order to prevent the degradation of the analytes. Once this time has passed, the samplers are collected and extracted immediately.

To do so, the sampler is removed from the water and the water on the outside is slightly dried. Taking care to not lose adsorbent, the contents are emptied into a centrifuge tube and, with the help of a few drops of water; those that remain adhered to the walls and cap of the sampler are dragged. The deuterated standards are added, and stirred so that they are adsorbed. It is centrifuged at 4000 rpm, for 5 minutes, the water is removed and the organic solvent is added (Table 2). It is stirred vigorously (vortex) for 1 minute and is sonicated for 10 minutes, in triplicate. It is centrifuged again (4000 rpm, 10min) and the supernatant is collected in an amber vial.

**Table 2: Experimental conditions for the different families of compounds that were studied.**

| Family | PLASTICISERS | CYTOSTATICS | PAH | PESTICIDES |
|---|---|---|---|---|
| No. of compounds | 5 | 15 | 15 | 11 |
| Water volume mL | 200 | 300 | 400 | 200 |
| ng standard | 2000 | 3000 | 400 | 1000 |
| Concentration µg L⁻¹ | 10 | 10 | 1 | 5 |
| Extraction solvent | MeOH | MeOH / MeOH:HCOOH (95:5) | DCM:Hexane (1:1) | DCM:Hexane (1:1) |
| Reconstitution solvent | MeOH/H2O (1:1) | ACN:HCOOH (0.1%) | n-Hexane | n-Hexane |
| | | H2O:HCOOH (0.1%) (1:1) | | |
| Reconstitution volume µL | 1000 | 500 | 500 | 500 |
| Analysis | LC-MS/MS | LC-MS/MS | GC-MS | GC-MS |
| Exposure time | 6 days | 6 days | 6 days | 6 days |

The extract is evaporated under a nitrogen current (TurboVap) up to a volume of approximately 1 mL. This volume is transferred to an injection vial, cleaning the amber vial with the same sample, and is dried (Reacti-Vap). It is reconstituted with the appropriate solvent (according to Table 1). This extract is analysed by gas chromatography coupled to mass spectrometry for volatile and non-polar compounds and by liquid chromatography coupled to mass spectrometry in tandem for the more polar and soluble compounds (Table 1).

The adsorbent is spiked with the native standards, inside the centrifuge tube, and is then extracted, evaporated and reconstituted just like the samples. These tests reveal the effectiveness of the extraction method for each family of contaminants.

In order to have an adequate performance, the kinetics for each type of compound must be studied. According to the bibliography, the retention capacity of the analytes is 1-40 ng/week.

## Claims

1. A passive ceramic sampler for measuring water contamination comprising a porous ceramic casing and an adsorbent filler material, **characterised in that** the ceramic casing has a microstructure that in turn comprises:
- a structural skeleton made of ceramic material in which spheroidal cavities with a diameter comprised between 10 and 15 µm are inserted, connected by windows smaller than 1 µm through which the liquid is permeated
- aggregates of the ceramic material that do not have cavities, but do have pores with sizes comprised between 20 and 90 nm.

2. The passive ceramic sampler according to claim 1, **characterised in that** the casing has a geometry that is selected among cylindrical, spherical, prismatic, ellipsoidal , toroidal, frustoconical or truncated-pyramidal.

3. The passive ceramic sampler according to claim 2, **characterised in that** the casing has a cylindrical geometry, with a length comprised between 10 and 300 mm, a diameter comprised between 2 and 100 mm and a thickness comprised between 0.5 and 5 mm, the ceramic material being alumina.

4. The passive ceramic sampler according to any one of claims 1 to 3, **characterised in that** the microstructure has an axial heterogeneity, the density of the cavities with a diameter comprised between 10 and 15 µm decreasing from the outside to the inside.

5. The passive ceramic sampler according to any one of claims 1 to 4, **characterised in that** all the pores of the microstructure of the casing have a diameter greater than 20 nm.

6. The passive ceramic sampler according to any one of claims 3 to 5, **characterised in that** the sampler with cylindrical geometry comprises caps in the bases in order to prevent the outlet of adsorbent material contained on the inside.

7. A method for the production of a passive ceramic sampler as defined in claims 1 to 6, **characterised in that** it comprises the following stages:
- preparing a dust suspension of the ceramic material with sizes comprised between 0.24 and 0.49 microns and water in proportions comprised between 60% and 80% by volume
- adding a pore-forming agent to the suspension in proportions comprised between 12% and 18% by volume and a dispersant with 1% by weight of the solid
- pouring the suspension prepared in the previous stages into a plaster matrix with the geometry of the desired end piece
- maintaining the suspension in the plaster matrix during a period of time comprised between 10 and 20 minutes
- sintering the ceramic material according to a cycle that in turn comprises:
- heating from room temperature to sintering temperature in a range between 1300 and 1500 °C with a slope of 4°C/min
- maintaining at the sintering temperature for 3 hours
- cooling to room temperature with a slope of 4°C/min

8. A use of a passive ceramic sampler as defined in claims 1 to 6 for detecting contaminants dissolved in water.

9. The use according to claim 8, wherein the contaminants are pesticides.

10. The use according to claim 8, wherein the contaminants are cytostatics.

11. The use according to claim 8, wherein the contaminants are polycyclic aromatic hydrocarbons.

12. The use according to claim 8, wherein the contaminants are plasticisers.
